# EUROPEAN PATENT APPLICATION

(11) **EP 2 233 575 A1**
(43) Date of publication of application: **29.09.2010**
(21) Application number: 07855702.2
(22) Date of filing: 18.12.2007
(51) Int. Cl.: C12N 15/51, C12N 15/85, C12N 15/86, C12N 15/861, C07K 14/18, A61K 48/00, A61P 1/16, A61P 31/14

(54) **AN ANTI-HCV VACCINE AND PREPARATION METHODS AND USES THEREOF**

(71) Applicant: Peking University People's Hospital, Beijing 100044 (CN)
(72) Inventor: WEI, Lai, Beijing 100044 (CN); TIAN, Yuan, Beijing 100044 (CN)
(74) Representative: Geyer, Fehners & Partner
(86) International application number: PCT/CN2007/003658
(87) International publication number: WO 2009/079834

(57) **Abstract**

An anti-HCV vaccine, which is prepared through recombination of an NS gene in series, including NS3/NS4 and NS4/NS5, with an adenovirus vector. The preparation methods and uses of the anti-HCV vaccine.

SEQUENCE LISTING

<110> Peking University People's Hospital

<120> AN ANTI-HCV VACCINE AND PREPARATION AND USES THEREOF

<130> KHP10312029.8

<160> 6

<170> PatentIn version 3.3

<210> 1

<211> 37

<212> DNA

<213> Artificial sequence

<220>

<223> Upstream primer for amplification of HCV NS3

<400> 1

atcctcgagc caccatggcg cccatcacgg cctactc 37

<210> 2

<211> 32

<212> DNA

<213> Artificial sequence

<220>

<223> Downstream primer for amplification of HCV NS3

<400> 2

atctctagac taagtgacga cctccaggtc ag 32

<210> 3

<211> 37

<212> DNA

<213> Artificial sequence

<220>

<223> Upstream primer for amplification of HCV NS4

<400> 3

atcctcgagc caccatgagc acctgggtgc tggtagg 37
<210> 4
<211> 32
<212> DNA
<213> Artificial sequence
<220>
<223> Downstream primer for amplification of HCV NS4
<400> 4
atctctagac tagcatggcg tagagcagtc ct 32
<210> 5
<211> 38
<212> DNA
<213> Artificial sequence
<220>
<223> Upstream primer for amplification of HCV NS5
<400> 5
atcgatatcg ccaccatgtc cggctcgtgg ctaaggga 38
<210> 6
<211> 29
<212> DNA
<213> Artificial sequence
<220>
<223> Downstream primer for amplification of HCV NS5
<400> 6
atctctagat catcggttgg ggagcaggt 29

## Description

### TECHNICAL FIELD

The invention belongs to the field of genetic engineering. It specifically relates to an anti-HCV vaccine, the preparation method and use thereof.

### BACKGROUND ART

The Hepatitis C virus (HCV) is a major public health problem. Around the world 1.7 million people are infected with HCV. In China, the standardized prevalence rate of HCV infection is about 3.2%. HCV infection is characterized by a high rate of chronicity, but chronic HCV infection is the major cause of liver cirrhosis, hepatocellular carcinoma and other major end-stage liver diseases.

To clear a patient of the virus can improve liver inflammation, inhibit liver fibrosis and prevent the progression of the disease. Interferon (IFN) α in combination with ribavirin (RBV) is the only relatively effective drug used in the treatment of HCV infection, but the sustained response rate (SVR) is low, and there are still some patients with no response or a relapse after treatment. Although pegylated interferon has been introduced and has increased SVR to 50-60%, a considerable number of patients still cannot obtain SVR after systematic treatment. Therefore, there remains an urgent need for people to find new HCV drugs and new treatment methods.

Lack of antigen-specific cellular and humoral immunity in chronic HCV patients may be the main reason for persistent infection with the HCV virus. The initiation of an immune response to a virus is mediated by the interaction between T cells and antigen-presenting cells (APC), while a specific immune response requires that antigen presenting cells efficiently uptake, process and present antigens to T lymphocytes. Meanwhile, T cells are activated through the stimulation of signaling molecules to activate a specific immune response, inhibit or clear the infection. Therefore, the study of HCV therapeutic vaccines, which can activate the specific immune response in chronic HCV infection patients and further clear the virus, may become the effective means for treatment of chronic hepatitis C.

At present, a research-focused immune response induced by an HCV peptide vaccine, is weak and restricted by the HLA type. As a result, it applies to a narrower group. In addition, the HCV structural gene expression was confirmed to have functions endangering DC. Therefore, the HCV non-structural gene should be the main candidate gene in preparing an HCV vaccine.

### SUMMARY OF INVENTION

Through the construction of a replication-defective adenovirus vector by recombination of various non-structural genes (NS genes) of HCV in series with an adenovirus, the inventor obtained ideal vaccines for the treatment of hepatitis C, thus laying the foundation for the immune therapy of chronic HCV infection.

The inventor found that the two genes in series induced a stronger cellular immune response than a single gene and multiple HCV epitope peptides combined immune response. Meanwhile, it overcame the deficiencies of tandem immunity by using more non-structural genes. The deficiencies include that amplification, transcription and translation errors are prone to occur, and it is hard to recombine due to the limited capacity of adenovirus vector.

Therefore, an objective of this invention is to provide a recombinant adenovirus vaccine for the treatment of hepatitis C; said vector vaccine respectively carries HCV non-structural genes, NS3/NS4 and NS4/NS5.

Another objective of this invention is to provide a method of preparing the vector vaccine carrying an HCV non-structural gene;

A further objective of this invention is to provide a use of the recombinant adenovirus vaccine in preparing the drugs used for treatment of HCV.

In order to achieve the objectives of this invention, the recombinant adenovirus vaccine of this invention is constructed as follows:
1. RNA is extracted from the serum of patients infected with HCV (type 1b), through RT-PCR HCV NS3, NS4, NS5, NS3/NS4 and NS4/NS5 genes are amplified. Gene amplification primers are listed in table 1 below:

| | Table 1: PCR primer | |
|---|---|---|
| Primer | Primer sequence | Location of target gene |
| NS3 upstream | 5' > ATC CTC GAG CCA CCA TGG CGC CCA TCA CGG CCT ACT C < 3' | 3351 nt-5177 nt |
| NS3 downstream | 5’ > ATC TCT AGA CTA    AGT GAC GAC CTC CAG GTC AG < 3’ | |
| NS4 upstream | 5' > ATC CTC GAG CCA CCA TGA GCA CCT GGG TGC TGG TAG G < 3' | 5178 nt-6371 nt |
| NS4 downstream | 5’ > ATC TCT AGA CTA GCA TGG CGT AGA GCA GTC CT < 3’ | |
| NS5 upstream | 5' > ATC GAT ATC GCC ACC ATG TCC GGC TCG TGG CTA AGG GA < 3' | 6372 nt-9362 nt |
| NS5 downstream | 5' > ATC TCT AGA TCA TCG GTT GGG GAG CAG GT < 3' | |

2. The HCV NS gene is cloned into Pshuttle-CMV plasmid. Double digestion of target gene and vector: HCV NS gene PCR products are extracted with phenol/chloroform, precipitated and recycled with ethanol, HCV NS3, NS4, NS3/NS4 PCR products and Pshuttle-CMV plasmid are double-digested with XbaI, XhoI, HCVNS5, NS4/NS5 PCR products and Pshuttle-CMV plasmid are double-digested with EcoRV, XhoI.

**Enzyme digestion system**

| | |
|---|---|
| 10x enzyme digestion buffer | 5µl |
| XhoI | 1µl |
| XbaI (or EcoRV) | 1µl |
| Target gene or plasmid | 5µl |
| Adding ddH₂O to the total volume | 50µl |

The target genes are ligated with the vector in a molar ratio of 3:1 with the help of T4 DNA ligase.
3. Pshuttle-CMV plasmids carrying the target gene and adenovirus backbone plasmid Adeasy1 (derived from adenovirus type 5, i.e. Ad5) are recombined in E.coli BJ5183, the correctly recombined max plasmids are identified with PacI incision enzyme, and prepared in a large scale.
4. Packaging, amplification and purification of adenovirus. adenovirus genomes are released by PacI digestion, 293 cells are transfected with liposome, the cells are collected after 7-10 days, repeatedly frozen and thawed, followed by CsCI density gradient centrifugation and taking the supernatant which contains purified infected adenovirus particles and has an adenovirus titer of 10¹¹PFU/ML.

The present invention compares the ability of the adenovirus vectors carrying different HCV NS genes and combined 7 HCV epitope peptides to induce cell immune response through animal experiments and in vitro human experiments, and selects HCVNS3/NS4 or NS4/NS5 with the highest immunogenicity as adenovirus vector vaccine of the target gene.

The invented viral vectors can be used in combination with other vaccine adjuvants, immunopotentiator that can be used includes: IL-12, unmethylated CpG motif of deoxyneucleotide or E. coli in DNA form, CaCl₂, PEG 6000 or Freund's excipient, etc..

For the purpose of clarity, the following embodiments and the drawings are provided to illustrate the present invention and should not be interpreted as limiting the scope of the invention.

### DESCRIPTION OF DRAWINGS

**Figure 1****:** illustrates electrophoresis of an HCV non-structural gene obtained through PCR.

Wherein, in diagram A, lanes 1 and 2 show the amplified results of NS3 and NS4, respectively; in digram B, lane 1 shows the NS5; in diagram C, diagram lanes 1 and 2, respectively, shows the amplified results of NS3/NS4 and NS4/NS5.

**Figure 2****:** illustrates the identification results of amplification of the target gene with adenovirus recombinant plasmid as a template.

**Figure 3****:** illustrates the identification results of digestion of the recombinant plasmid with PacI incision enzyme.

**Figure 4****:** illustrates the identification results of HCV NS protein expression.

### SPECIFIC MODE OF CARRYING OUT THE INVENTION

The present invention will be further illustrated in combination with specific examples. It should be understood that these examples are only intended to explain the present invention without limiting the scope of protection of the invention.

### Example 1

Construction of an adenovirus vector carrying various HCV non-structural genes and identification of an HCV protein expression.

### 1. Experimental Materials

HCV infected serum was derived from volunteer donors, informed consent was signed.

Pshuttle-CMV (Germany, Merck, No. ST240007), Adeasy-1 plasmid (Germany, Merck, No. ST240005), E. coli BJ5183, and the recombinant adenovirus carrying GFP (AdGFP) were purchased from Beijing Nuosai genome Research Center Co., Ltd.;

Huh7 hepatoma cells were purchased from the People's Hospital (JIA Yintang, etc. construction of interferon stimulated gene ISG20 eukaryotic expression vector and study of its anti-hepatitis C virus replication, the Chinese Journal of Immunology, 2006 Vol.22 No.11 P. 997-1001); DH5α chemical conversion competent was purchased from Beijing Ding States Biotechnology Co., Ltd.;

M-MLV Rtase cDNA synthesis Kit and LA-Taq DNA polymerase were purchased from Japan TOKARA Company;

XbaI, XhoI, EcoRV, PmeI, PacI restriction enzymes, T4 DNA ligase and calf intestinal alkaline phosphatase (CIAP) were purchased from Promega Corporation;

RNA extraction kit (QIAGEN Rneasy Mini kit), DNA gel recycle and purification kit (QIA quick Gel Extraction Kit) and Maxi plasmid Extract Kit were purchased from Qiagen Company, Germany;

PCR primers were designed with reference to HCV1b sequence with the introduction of restriction sites, synthesized by the Beijing Saibaisheng Gene Technology Co., Ltd., primer sequences and amplified fragment length are listed in table 1;

Cell culture reagents: DMEM (Dulbecco's modified Eagle's medium) medium and fetal bovine serum (fetal bovine serum, FBS) were purchased from Hyclone, Inc.;

DMSO (dimathyl sulfoxide, DMSO) was purchased from Sigma Company;

Trypsin and EDTA (ethylenediamine tetraacetic acid, EDTA) were purchased from Sino-American Biotechnology Company;

Antibodies: mouse anti-HCV NS3, anti-HCV NS4A and anti-HCV NS5A monoclonal antibody were purchased from the United States BIODESIGN company;

HRP labeled goat anti-mouse IgG were purchased from Santa Cruz, Inc.; Agar, yeast extract, tryptone were purchased from Oxid Company;

Agarose was purchased from Flua Company.

### 2. Experimental Methods

1) RT-PCR amplification of HCV NS3, NS4, NS5, NS3/NS4, NS4/NS5 gene:
   RNA was extracted from HCV1b type infected serum (method see QIAGEN Rneasy Mini kit instructions), the cDNA was obtained by reverse transcription using downstream primers of the target genes (see Table 1) (method see the M-MLV Rtase cDNA synthesis Kit manual), various HCV NS genes were obtained through PCR amplification with this cDNA as a template.
   PCR reaction conditions:
      HCV NS3, NS4, NS3/NS4: pre-denaturing at 94 °C for 5min, denaturing at 94 °C for 1min, annealing at 55 °C for 1min, extending at 72 °C for 3min, 30 cycles in total.
      HCV NS5 and NS4/NS5: pre-denaturating at 94 °C for 5min, denaturing at 94 °C for 1min, annealing at 58 °C for 1min, extending at 72 °C for 4min, 30 cycles in total.

**PCR reaction system**

| | |
|---|---|
| 10xPCR buffer solution | 5µl |
| dNTP(10mM) | 0.5µl |
| Target gene primer (2.5pM upstream | 1µl, respectively |
| and downstream, respectively) | |
| Template plasmid | 1µl |
| LA-Taq DNA polymerase | 0.5µl |
| Adding ddH₂O to total volume | 50µl |

2) Cloning HCV NS gene into Pshuttle-CMV plasmid Double digestion of the target gene and the vector: HCV NS gene PCR products were extracted with phenol/ chloroform, precipitated and recycled with ethanol, HCV NS3, NS4, NS3/NS4 amplification products and Pshuttle-CMV plasmid were double-digested with XbaI, XhoI; HCVNS5, NS4/NS5 amplification products and Pshuttle-CMV plasmid are double-digested with EcoRV, XhoI.

**Enzyme digestion system**

| | |
|---|---|
| 10x enzyme digestion buffer | 5µl |
| XhoI | 1µl |
| XbaI (or EcoRV) | 1µl |
| Target gene or plasmid | 5µl |
| Adding ddH₂O to total volume | 50µl |

Following digestion at 37°C for 2 hours and gel extraction with 0.7% agarose gel electrophoresis, the digested plasmids and target fragments were recovered with the DNA gel recovery kit and then dissolved in 8µl H₂O, 3µl of which was taken to undergo quantitative agarose electrophoresis

The ligation of the target gene and the vector:
Target gene was ligated with the vector in a molar ratio of 3:1.

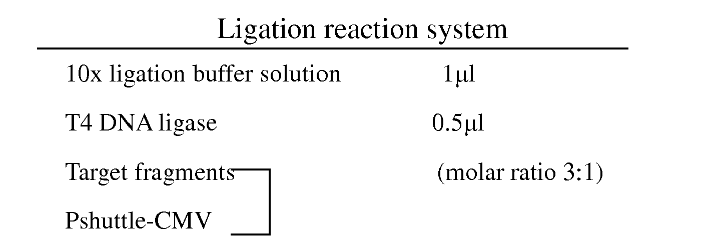

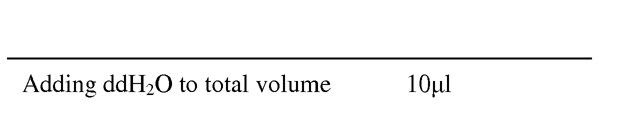

Ligating at 16°C overnight

### 3) Transformed into E. coli and identification of positive clones

E. coli chemosensory peptide 50µl was gently mixed with 5µl ligation product, kept in an ice bath for 30min, then underwent heat shock at 42°C for 90s and was kept in an ice bath for an additional 2min, followed by adding 1ml of LB medium and incubating it at 37°C for 1 hour under constant temperature shaking at 180rpm. The bacteria were evenly distributed on the kanamycin-resistent LB plate, the plate was kept at room temperature until the liquid was absorbed, and the plate was inverted and incubated at 37°C for 16 hours. 15 monoclonal colonies were picked up with a toothpick and inoculated in 2ml of LB medium containing kanamycin, incubated overnight at 37 °C while shaking (230rpm). The small extracted plasmids were then incubated, the steps are provided as follows:

| |
|---|
| Transferring 2ml of bacteria in the tube into EP tube |
| ↓ |
| Centrifuging at 12000rpm at 4°C for 1min |
| ↓ |
| Removing the supernatant, adding 100µl of basic lysis solution I , shaking sharply |
| ↓ |
| Adding 200µl of room temperature basic lysis solution on ice, inversing |
| ◄ |
| rapidly and mixing evenly |
| ↓ |
| Adding 150µl of cold room temperature basic lysis solution on ice, mixing evenly and kept in an ice bath for 3-5min |
| ↓ |
| Centrifuging at 12000rpm at 4°C for 5mim |
| ↓ |

Transferring the supernatant into another EP tube , adding twice the volume of ethanol to precipitate nucleic acid under room temperature The obtained plasmids were identified through double digestion and PCR. The method was the same as stated above.

### 4) Preparation of electrotransformed competent E. coli BJ5183

The steps are provided as follows:
500µl of BJ5183 bacteria was inoculated in 50ml of LB cuture solution ,

| |
|---|
| 37°C, 250rpm overnight |
| ↓ |
| Inoculating 25ml of overnight culture in duplicate into 500mlLB culture solution at 37 °C, 250rpm |
| ↓ |
| When OD₆₀₀ is 0.4 , keeping the culture in an ice bath for 15-30min |
| ↓ |
| Centrifuging at 4°C, 2500 rpm for 20min , removing the supernatant |
| ↓ |
| Resuspending with 250ml of cold 10% glycerol , centrifuging at 4°C, 2500 rpm for 20min , removing the supernatant |
| ↓ |
| Repeating the process three times, maintaining 2ml of supernatant in the last time , resupending the bacteria |
| ↓ |
| Split charging the competent cells, merging them into liquid nitrogen for sharp freezing, storing at -70 °C for reserve |

### 5) Recombination of Pshuttle-CMV plasmid carrying target genes and adenovirus backbone plasmid Adeasy1

Linealization and dephosphorylation of Pshuttle-CMV plasmid carrying target genes:

**Enzyme digestion reaction system**

| | |
|---|---|
| 10x enzyme digestion buffer | 5µl |
| solution | |
| Pmel endonuclease | 1µl |
| Target gene or plasmid | 5µl |
| Adding ddH₂O to total volume | 50µl |

After reacting at 37°C for 2 hours , complete linearization was tested through agarose gel electrophoresis , the linearized fragments were cut and recovered , the dephosphorylation was performed using CIAP.

**Dephosphorylation reaction system**

| | |
|---|---|
| AP buffer | 10µl |
| CIAP | 2µl |
| ddH₂O | 80µl |
| Linearized Pshuttle | 8µl |

50°C, 15min, 37°C, 15min, followed by supplementing 2µlCIAP, again, 50°C, 15min, 37°C, 15min, extrating with phenol/chloroform and precipitating with ethanol for reserve.

BJ5183 was transformed with both Pshuttle-CMV and Adeasy1: 40µlBJ5183 electrotnic transfer competence was taken, while adding 2µl linearized and dephosphorylated Pshuttle-CMV and Adeasy1, respectively, (15ng or so), followed by placing on ice for 60s. Electroporator was regulated with electric pulse 25µF, voltage 2.5kV, and resistance 200Ω. Mixture of bacteria and plasmid was added to electrical rotor, the pulse on bacteria was started by set parameters. After completion, 1ml LB medium was quickly added , and then cultured at 37 °C, 180rpm for 1 hour. The culture was spread on ampicillin and kanamycin double-resistant LB plate. while the liquid is absorbed, the culture plate was inversed and incubated at 37°C.

Identification of recombinants: monoclonal colonies were picked and inoculated into 2ml LB media, and shaken overnight. The plasmids were extracted in a small amount, and the plasmids recombined correctly were identified using PacI endonuclease.

Large scale preparation of the correctly recombined adenovirus plasmids: Max plasmid kit was used, the method referred to in the manual.

### 6) The packaging, amplification and purification of adenovirus Adenovirus genome was released though PacI digestion, 293 cells was transfected with liposome, after 7-10 days the cells were collected, followed by repeatedly freeze-thawing at -70 °C and room temperature, CsCl density gradient centrifugation. The obtained supernatant contained infectious adenovirus particles. The adenovirus can be amplified by infecting 293 cells multiple times.

### 7) Incubation of Huh7 Cells

Human hepatoma cell line Huh7 cells were inoculated in 6cm culture dishes in 1.5x10⁶, the medium was DMEM containing 10% FBS, 200µmol L-glutamine, 100IU/ml penicillin and streptomycin, which was placed in a cell incubator containing 5% CO₂ at 37°C. After 24 hours, the culture solution was removed, recombinant adenovirus carrying HCV NS to was added in complex infection index (MOT) 50. 1.5 hours later, the medium was added, and incubated at 37°C, 5% CO₂ for further 48 hours.

### 8) The extraction of total cellular protein

The culture solution was removed, after washing 2 times with PBS, 80 µl of cell lysate was added. The Cells were scraped with cell scraper, collected in an EP tube and kept in an ice bath for 30 min. After 4°C, 12000 rpm centrifugation for 20 minutes, the obtained supernatant is total cellular protein lysate. The concentration of total cellular protein was determined through BCA, and then stored at -70°C.

### 9) Western Blot detection of HCV NS protein expression

### 3. Experimental results:

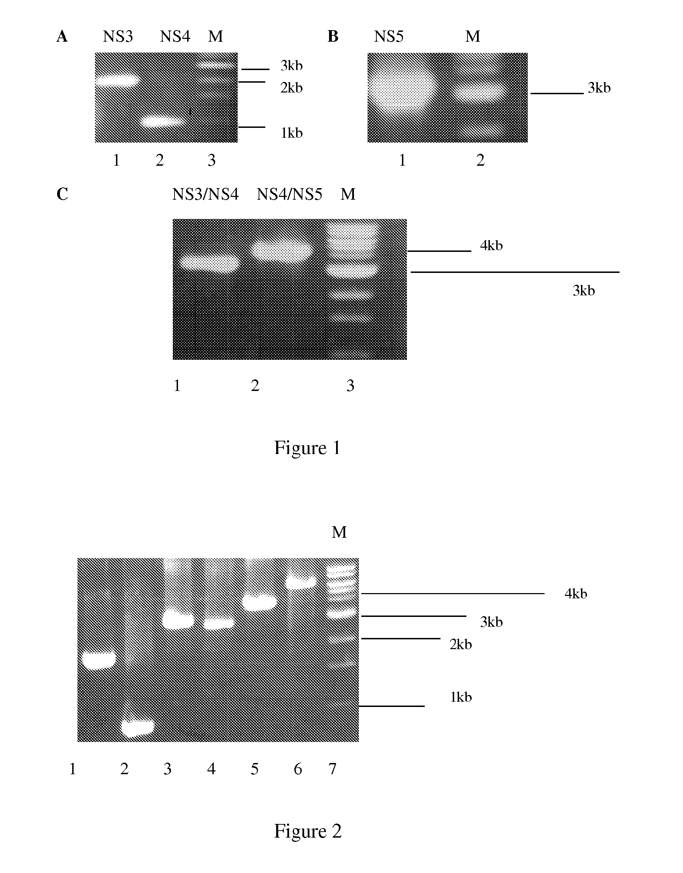

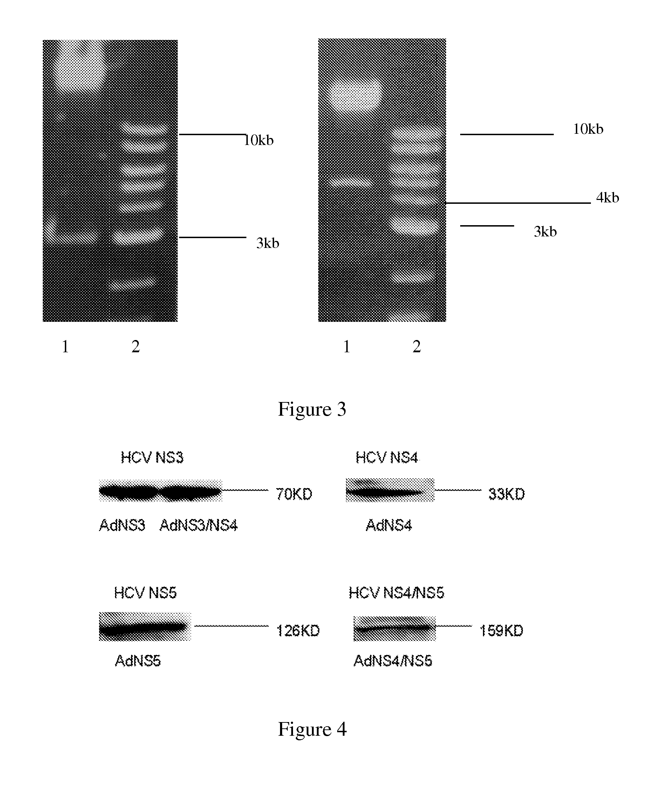

1) The above genes were successfully obtained through the PCR method; the size of fragments is 1826bp, 1193bp, 2999bp, 3019bp, 4192bp, respectively. The electrophoresis results are shown in Figure 1.
2) Correctly recombined adenovirus plasmids carrying various HCV NS genes. The recombinant plasmids were identified through PCR, see Figure 2, in which lane 1 shows HCV NS3 (1.8kb), lane 2 shows HCV NS4 (1.2kb), lane 3 shows HCV NS5 (3kb), lane 4 shows HCV NS3/NS4 (3kb), lanes 5 shows HCV NS4/NS5 (4kb); The recombinant plasmids were identified through enzyme digestion with PacI endonuclease, correctly recombined max plasmids are digested by PacI to release small fragments of 4.5kb or 3kb, and large fragments of above 30kb (Figure 3).
3) The target cells transfected by the constructed recombinant adenovirus can express the HCV target protein correctly. As shown in Figure 4, 70KD, 33 KD, 126 KD, 159 KD bands were HCV NS3, NS4, NS5, NS3/NS4 and NS4/NS5 protein respectively.

### Example 2: In vitro experiments for healthy subjects

### 1. Experimental Materials

1) Subjects: peripheral blood of 19 HLA-A2-positive health subjects was derived from the Beijing Red Cross Blood Center.
2) Lymphocyte Separating Solution: purchased from Tripod State Biotechnology Co., Ltd., the specific gravity is 1.077g/ml;
   Recombinant human granulocyte-macrophage colony-stimulating factor (rh GM-CSF) and recombinant human interleukin-4 (rh-IL-4) were purchased from American R & D companies;
   AIM-V in serum-free culture medium was purchased from GIBCO. Inc.;
   RPMI 1640 medium was purchased from Biotech Co., Ltd. Ding States. Complete RPMI 1640 contains 10% fetal bovine serum, 1% Glutamine, 100IU/ml streptomycin and 100µg/ml penicillin;
   Mouse anti-human HLA-A2 monoclonal antibody was purchased from American BD Company. LPS and mitomycin C were purchased from American Sigma Company;
   Hhu7 cells (HCVR) which were stably transfected with HCV replicon and can express HCV NS3, NS4 and NS5 proteins were purchased from People's Hospital (JIA Yintang etc., Inhibition of interferon-α on hepatitis C virus replicon, Chinese Medical Journal, 2005 Vol .85 No.29 P.2065-2069) and identified as HLA-A2 positive;
   IFN-γ, IL-4, and GrB ELISPOT assay kits were purchased from France DIACLONE Company;
   IL-2 was purchased from an American R & D Company;
   7 HLA-A2 restriction CTL epitope polypeptides originated from HCV NS3, NS4 and NS5 region, and were synthesized by Shanghai Sangon. After purification through high performance liquid chromatography (HPLC), it has a purity of more than 90%. its freeze-dried powder was dissolved in DMSO with 5mg/ml concentration, then diluted to 1mg/ml with PBS, filled separately, and cryostored at -20°C for reserve. The peptide sequences are shown in Table 2.

**Table 2 Origin and Sequence of HLA-A2 restriction CTL epitope Polypeptide**

| Polypeptide No. | Viral protein | Amino acid site | Amino acid sequence |
|---|---|---|---|
| P1 | HCV NS3 | 1073-1081 | CINGVCWTV |
| P2 | HCV NS3 | 1406-1415 | KLVALGINAV |
| P3 | HCV NS4B | 1671-1680 | VLAALAAYCL |
| P4 | HCV NS4B | 1807-1816 | LLFNILGGWV |
| P5 | HCV NS5A | 2145-2154 | LLREEVSFRV |
| P6 | HCV NS5B | 2578-2587 | RLIVFPDLGV |
| P7 | HCV NS5B | 2594-2602 | ALYDVVTKL |

### 2. Experimental methods

### 1) Identification of HLA-A2 type

Whole blood method: Whole blood: 200 ul of fresh collected anticoagulant peripheral whole blood was divided into two: The sample tube and negative control tube, 100 ul /each tube, then 2ml erythrocyte lysis buffer was added, followed by flicking tube to mix evently, treating 10min and then centrifuging(1500rpm, 4°C, 5min). The supernatant was removed, the cells were washed twice with FACS wash solution, in which, after the experimental tube was added 100 ul of mouse anti-human HLA-A2 antibody (HB8.2 cell strain culture supernatant) was incubated at room temperature for 15min in the same way as the control tube. The cells were washed twice with FACS wash solution; in each tube 200ul FACS fixative was added for flow cytometry detection. CELLQuest software was used to analyze test results.

### 2) Separation of peripheral blood mononuclear cells (PBMC) through density gradient centrifugation

Collected peripheral venous blood was anticoagulated with EDTA-K3. The whole blood was slowly added along the wall to a sterile centrifuge tube containing an equal amount of Ficoll-Hypaque layered liquid, and centrifuged at room temperature(1500rpm, 15 ∼ 18min). A mononuclear cell layer was drawn with a sharp straw. The cells were washed three times with serum-free RPMI1640 (pH 7.2), and then centrifuged at room temperature for 10min, at 1500 rpm, 1200 rpm and 1000 rpm, respectively. Complete RPMI1640 medium was used to suspend cells and adjust the cell concentration to 2 × 10⁶/ml, the cells were added to 6 well plates, 2ml per hole, and incubated in an 37°C, 5% CO₂ incubator for 4h to allow the monocytes to adhere to the wall. The culture plate was gently washed with warm serum-free RPMI1640 medium to remove non-adherent and to obtain adherent monocytes for induction of DC. Non-adherent cells were collected: cryostored at -70°C for reserve.

### 3) In vitro amplification and identification of dendritic cells

Adherent monocytes were incubated in reference to the method of Romani N (Romani N, Gruner S, Brang D, et al. , Proliferatin dendritic cell progenitors in human blood.J Exp Med, 1994,180 (1): 83-93). AIM-V medium containing rh GM-CSF1000IU/ml, rhIL-4 800IU/ml were added to the culture plate and placed in 37°C, 5% CO₂ incubator, a half-volume of culture fluid was replaced every other day, suspension cells were collected on the 7^{th} day. Morphology was observed by light microscopy. The collected DC was transferred into flow cytometry tubes, 1 × 10⁵ / tube, suspended with FACS wash liquid, followed by centrifugation and removal of supernatant. To tube FITC labeled mouse anti-human CD83, CD86, CD14 monoclonal antibody and PE labeled mouse anti-human HLA-DR, CD1a, CD11c, CD80 monoclonal antibody 5µl were added, respectively. After labeled at room temperature and kept away from light for 15min, the cells were washed twice with FACS wash liquid. To each tube was added 200µlFACS fixative; the cell phenotype was detected by flow cytometry.

### 4) Adenovirus infection

DC originated from peripheral blood of five healthy individuals was infected in MOI400 with AdNS3, AdNS4 and AdNS5, respectively, on the sixth day. AdGFP infected DC (MOI400) was set as the control group A; DC originated from peripheral blood of five healthy individuals was infected in MOI400 with AdNS3, AdNS4 and AdNS3/NS4, respectively, on the sixth day, set as group B; DC derived from other four human peripheral blood was infected in MOI400 with AdNS4, AdNS5 and AdNS4/NS5, respectively, set as C; the remaining six healthy human derived DC was infected with AdNS3/NS4 and AdNS4/NS5, AdGFP infected DC (MOI400) as the control, set D, in the group D, part of the DC was left uninfected, while the infected DC was collected, the uninfected DC was collected. Uninfected DC was simultaneously loaded with 7 HLA-A2 restriction CTL epitope polypeptides for 2 hours, the final concentration was 10 µg / ml.

### 5) The effect of mitomycin

To HCV gene transfected DC and peptide loading DC was added 50 µg / ml MMC, respectively, after reacting at 37°C for 45 min, fully washed with medium to remove residual MMC, and then added to 96-well bottom culture plates with cell number of 2 × 10⁴ / well.

### 6) Interaction between non-adherent PBMC and DC

Recovered cryostored homologous non-adherent PBMC was added to the DC culture well in the 1:10 ratio in triplicate. After 4 days, IL-2 30U/ml was added, the half-volume medium was replaced every other day, following incubation for further 3 days, T cells were harvested and counted.

### 7) Detection of the frequency of T-cell secreting IFN-γ, IL-4 and GrB.

DIACLONE's IFN-γ, IL-4, and GrB ELISPOT kits were used, methods refered to instructions, HCVR was used as the target cell or secondary stimulus, the effector-target ratio is 10:1.

### 3. Experimental results (see Table 3, 4, 5, 6)

1) Cellular immune responses induced by the adenovirus vector carrying HCVNS3, NS4 and NS5 gene showed no significant difference.
2) Cellular immune responses induced by the adenovirus vector carrying HCVNS3/NS4 gene were stronger than those induced by adenovirus vector carrying the HCVNS3 or HCVNS4 gene.
3) Cellular immune responses induced by the adenovirus vector carrying an HCVNS4/NS5 gene were stronger than those induced by adenovirus vector carrying HCVNS4 or HCVNS5 gene.
4) Cellular immune responses induced by the adenovirus vector respectively carrying HCVNS3/NS4 and HCVNS4/NS5 gene showed no significant difference, and were stronger than those induced by 7 peptides combined loading DC.

**Table 3 Results of IFN-γ, IL-4 and GrB ELISPOT in group A**

| (SFC/2x10⁵PBMC) | | | | |
|---|---|---|---|---|
| | AdNS3 | AdNS4 | AdNS5 | AdGFP |
| IFN-γ | 158.2±55.94 | 113.6±23.42 | 126.8±40.52 | 39.8±12.74 |
| IL-4 | 92.4±40.04 | 58.4±12.58 | 72.0±13.27 | 21.6±9.81 |
| GrB | 220.43±50.15 | 194.0±52.08 | 211.2 ±57.12 | 59.4±16.77 |

**Table 4 Results of IFN-γ, IL-4 and GrB ELISPOT in group B (SFC/2x10⁵PBMC)**

| | AdNS3 | AdNS4 | AdNS3/NS4 |
|---|---|---|---|
| IFN-γ | 157.75±36.45 | 101.75±25.05 | 212.0±44.66 |
| IL-4 | 84.75±17.86 | 63.5±10.63 | 125.5±29.59 |
| GrB | 193.25±26.66 | 184.0±44.21 | 292.75±25.55 |

**Table 5 Results of IFN-γ, IL-4 and GrB ELISPOT in group C (SFC/2x10⁵PBMC)**

| | AdNS3 | AdNS4 | AdNS3/NS4 |
|---|---|---|---|
| IFN-γ | 157.75±36.45 | 101.75±25.05 | 212.0±44.66 |
| IL-4 | 84.75±17.86 | 63.5±10.63 | 125.5±29.59 |
| GrB | 193.25±26.66 | 184.0±44.21 | 292.75±25.55 |

**Table 6 Results of IFN-γ, IL-4 and GrB ELISPOT in group D**

| (SFC/2x10⁵PBMC) | | | | |
|---|---|---|---|---|
| | Ad NS3/NS4 | Ad NS4/NS5 | CTL 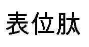 | AdGFP |
| IFN-γ | 242.17±36.44 | 258.17±28.13 | 156.67±14.24 | 46.33±8.2 |
| IL-4 | 92.33±30.65 | 86.5±21.10 | 36.83±9.47 | 20.33±6.2 |
| GrB | 260.5±64.70 | 275.83±59.28 | 145.67±19.04 | 57.83±9.1 |

### Example 3: In vitro experiments of HCV-infected patients

### 1. Experimental Materials

As stated above

### 2. Experimental Methods

HCV-infected group was treated the same as group D in example 2 was, when non-adherent PBMC and DC interacted, they first interacted for 24 hours before adding IL-2 30IU/ml, half-volume medium was replaced every other day, and then the culture was incubated for a further 48 hours. Other steps were the same as stated above.

### 3. Experimental results (see Table 7)

Adenovirus vectors carrying HCVNS3/NS4 and HCVNS4/NS5 genes all induced strong cellular immune responses, but there was no significant difference between the two, and also they induced stronger immune responses than 7 peptides combined loading DC did.

**Table 7 the results of IFN-γ , IL-4 and GrB ELISPOT in HCV-infected group (SFC/2x10⁵PBMC)**

| | Ad NS3/NS4 | Ad NS4/NS5 | CTL epitope peptidep | AdGFP |
|---|---|---|---|---|
| IFN-γ | 176.75±23.46 | 185.25±23.11 | 116.75±20.45 | 44.75±11.64 |
| IL-4 | 98.0±12.36 | 53.5±15.86 | 38.25±8.14 | 22.75±3.86 |
| GrB | 298.75±51.12 | 301.5±92.44 | 175.25±31.27 | 56.25±6.65 |

### Example 4: Animal experiments

### 1. Experimental Materials and Experimental Methods

Experimental animals were 18 female Balb/c mice 5-6 weeks old, which were randomly divided into three groups, and intraperitoneally injected with 1x10⁸PFU recombinant adenovirus carrying HCVNS3/NS4, HCVNS4/NS5 and PBS, respectively. The mice were then injected once every other 12 days, three times in total. 12 days after the third injection, the mice were killed. Their spleens were taken under aseptic conditions and grinded to form single-celled suspension for IFN-γ ELISPOT experiment, still HCVR was used as the target cells, and the method was the same as stated above.

### 2. Experimental results (see Table 8)

Adenovirus carrying HCVNS3/NS4 or HCVNS4/NS5 genes all can induce a strong cellular immune response, and no significant difference was found between the two.

**Table 8 the results of IFN-γELISPOT in animal experiment (SFC/5x10⁵PBMC)**

| | AdNS3/NS4 | AdNS4/NS5 | PBS |
|---|---|---|---|
| IFN-γ | 101.32±13.33 | 92.89±10.67 | 16.46±7.90 |

### INDUSTRIAL APPLICABILITY

According to the method described in the present invention, the anti-HCV vaccine is prepared through recombination of the HCV NS genes combined in series with the adenovirus vector, or further combined with an immunopotentiator to form anti-HCV drugs for prevention and treatment of chronic HCV infection. Animal experiments show that the present adenovirus anti-HCV vaccine carrying HCVNS3/NS4 or HCVNS4/NS5 genes can induce a strong cellular immune response, and it is safe, easy to use and free from intramuscular injection and other specific conditions.

## Claims

1. An anti-HCV vaccine, which is prepared through recombination of NS genes in series with an adenovirus vector.

2. The vaccine as claimed in claim 1, wherein the said NS genes in series are NS3/NS4 or NS4/NS5.

3. The vaccine as claimed in claim 1 or 2, wherein the said adenovirus is replication-defective Ad5.

4. A method of preparing the vaccine claimed in any of claims 1-3, including the following steps:
1) Preparation of the target genes :designing primers, obtaining HCV NS genes through RT-PCR;
2) Cloning HCV NS genes into Pshuttle-CMV plasmid;
3) Ligating the target genes and vectors in molar ratio of 3:1;
4) Recombining the Pshuttle-CMV plasmid carrying the target genes with adenovirus backbone plasmid Adeasy1;
5) After enzyme digestion, transfection, inoculating cell 293, freeze thrawing 2-3 times, CsCl density gradient centrifugation, cryopreserving the purified recombinant adenovirus with a uniform genome structure.

5. The method as claimed in claim 1 , the amplified primers through RT-PCR are the following:
| Primer | Primer sequence | Location of target gene |
|---|---|---|
| NS3 upstream | 5' > ATC CTC GAG CCA CCA TGG CGC CCA TCA CGG CCT ACT C < 3' | 3351 nt-5177 nt |
| NS3 downstream | 5’ > ATC TCT AGA CTA    AGT GAC GAC CTC CAG GTC AG < 3’ | |
| NS4 upstream | 5' > ATC CTC GAG CCA CCA TGA GCA CCT GGG TGC TGG TAG G < 3' | 5178 nt-6371 nt |
| NS4 downstream | 5' > ATC TCT AGA CTA GCA TGG CGT AGA GCA GTC CT < 3' | |
| NS5 upstream | 5' > ATC GAT ATC GCC ACC ATG TCC GGC TCG TGG CTA AGG GA < 3' | 6372 nt-9362 nt |
| NS5 downstream | 5' > ATC TCT AGA TCA TCG GTT GGG GAG CAG GT < 3' | |

6. A use of the vaccine as claimed in any of claims 1-3 in preparing the drugs used for treatment of HCV.

7. The use as claimed in claim 6, wherein the said drugs can be a mixture of vactor vaccine and immunopotentiator.

8. The use as claimed in claim 6 or 7, wherein the said immunopotentiator can be one or more selected from the group consisting of IL-12, unmethylated CpG motif of deoxyneucleotide or E. coli in DNA form, CaCl₂, PEG 6000 or Freund's excipient.
